Europäisches Patentamt

(19) European Patent Office

Office européen des brevets

(11) Publication number: **0 086 228**
**B1**

(12) **EUROPEAN PATENT SPECIFICATION**

(45) Date of publication of patent specification: **11.05.88**

(51) Int. Cl.⁴: **A 61 K 31/71,** A 61 K 31/65, A 61 K 31/455, A 61 K 31/17

(21) Application number: **82902867.9**

(22) Date of filing: **25.08.82**

(86) International application number: **PCT/US82/01153**

(87) International publication number: **WO 83/00628 03.03.83 Gazette 83/06**

(54) **USE OF CARBAMIDE PEROXIDE FOR THE MANUFACTURE OF A MEDICAMENT FOR TREATMENT OF ACNE VULGARIS.**

(30) Priority: **27.08.81 US 296920**

(43) Date of publication of application:
**24.08.83 Bulletin 83/34**

(45) Publication of the grant of the patent:
**11.05.88 Bulletin 88/19**

(84) Designated Contracting States:
**AT BE CH DE FR GB LI LU NL SE**

(56) References cited:
**GB-A- 964 444**
**US-A-2 430 450**
**US-A-3 657 413**
**US-A-4 302 441**

**Handbook of Nonprescription Drugs, 5th Ed. Published January 1977, See page 319**

The file contains technical information submitted after the application was filed and not included in this specification

(73) Proprietor: **SOFT SHEEN PRODUCTS, INC.**
**1000 East 87th Street**
**Chicago, IL 60619 (US)**

(72) Inventor: **BERNSTEIN, Joel E.**
**615 Brierhill Road**
**Deerfield, IL 60015 (US)**

(74) Representative: **Baillie, Iain Cameron et al**
**c/o Ladas & Parry Isartorplatz 5**
**D-8000 München 2 (DE)**

## Description

The present invention relates to a composition for treating acne vulgaris.

Acne vulgaris is an inflammatory disease of the pilosebaceous glands characterized by an eruption of the skin, often pustular in nature but not suppurative. Acne is a common affliction of the adolescent and affects a small but significant percentage of the adult population. Acne lesions are of four basis types: comedones (blackheads or whiteheads), papules, pustules, and cysts (or nodules). Various topical agents are utilized in the treatment of acne and these include sulfur, resorcinol, salicylic acid, benzoyl peroxide, vitamin A acid and topical antibiotics. Acne involvement results in unsightly lesions, particularly on the face, and in some cases results in severe scarring.

There are a variety of methods for treating acne vulgaris including topically applying various scrubbing or abrasive compositions, topically applying deep cleaning or astringent compositions and also exposure to ultraviolet radiation. Nevertheless, acne vulgaris is seldom cured and only can be contained with difficulty.

European Patent Publication No. 10437 discloses a composition for treating acne vulgaris which includes erythromycin as the active ingredient. British Patent No. 964,444 discloses compositions for treatment of acne vulgaris one of which includes esters of nicotinic acid.

U.S. Patent No. 3,657,413 relates to an antiseptic composition including urea peroxide, glycerol and a carboxypolymethylene polymer.

Carbamide peroxide is an agent used to soften earwax for removal.

The object of the present invention is to provide an improved composition for treatment of acne vulgaris.

The present invention provides a composition effective in decreasing the inflammatory lesions of acne vulgaris in human patients having such inflammatory lesions comprising a carrier containing a therapeutically effective amount of carbamide peroxide in combination with a therapeutically effective amount of nicotinic acid or nicotinamide.

The present invention also provides the use of a therapeutically effective amount of carbamide peroxide for the manufacture of a topical ointment, cream or lotion for the treatment of acne vulgaris including open and closed comedones.

Topical formulations containing carbamide peroxide have been tested for effectiveness and found to be useful in the treatment of acne vulgaris. Also surprisingly carbamide peroxide achieves such beneficial effects without much of the irritating and sensitizing effects of benzoyl peroxide. It has also been found that combinations of carbamide peroxide with certain chemical agents known to be effective in treating acne are more effective in treating acne then would be expected by treatment with the individual agents themselves. Such formulations include combinations of carbamide peroxide and nicotinic acid or nicotinamide.

In the practice of this invention, topical solutions of carbamide peroxide in various organic vehicles such as a combination of ethyl alcohol and propylene glycol in which the active ingredient is present in the range of from 1% to 15% by volume of the carrier are prepared.

Additionally topical solutions of carbamine peroxide in various organic carriers in concentrations ranging from 1 to 15% by volume of the carrier are incorporated into various organic vehicles including solutions, lotions, creams, gels and ointments along with one or more of the following ingredients: nicotinic acid or nicotinamide in concentrations of from 1% to 10% by volume of the carrier. Such carriers useful for the incorporation of carbamide peroxide include combinations of ethyl alcohol and propylene glycol as well as surface active agents such as lauryl ethers and lauryl esters. Applications of the carrier and effective ingredient are made to the face of acne patients 2 to 4 times daily with the result that open and closed comedones (blackheads and whiteheads) are markedly reduced within two weeks. The following examples illustrate the present invention.

### Example 1

A 1% solution of carbamide peroxide was prepared in an alcohol-propylene glycol carrier. Twice daily topical application of this solution were self-administered by a 26 year old female patient suffering from acne vulgaris. After two weeks of treatment the comedone count on the patient's face had declined from 28 to 15.

### Example 2

A 20 year-old male applied 10% carbamide peroxide prepared in an alcohol gel containing 6% polyoxyethylene lauryl ether four times daily. After 10 days the number of comedones on his face had declined from 43 to 25 and by the end of four weeks of treatment he had only 18 comedones on his face.

### Example 3

A 30 year-old female with acne vulgaris applied a 5% by volume solution of carbamide peroxide in a 70% ethyl alcohol and 30% propylene glycol carrier. The product was applied to all involved areas of the face and back twice daily. Before treatment this patient had 64 comedones on the face and back but after two weeks of treatment she had only 41 comedones in these areas.

### Example 4

A 2.5% carbamide peroxide cream was prepared containing water, propylene glycol, bentonite, glycerol stearate, isopropyl myristate and cellulose gum and applied twice daily to the skin of a 24 year-old male with many comedones on his forehead. After two weeks of such treatment the number of comedones on his forehead had declined from 28 to 12.

Example 5

A 26 year-old male who had been unable to use 5 or 10% benzoyl peroxide preparations due to rashes each time he used them, applied a topical 5% carbamide peroxide preparation twice daily for eight weeks. The number of comedones declined over this treatment peroid from 46 to 29 at two weeks and to only 16 comedones after eight weeks of therapy.

Example 6

A alcohol-propylene glycol vehicle containing 5% carbamide peroxide and 2% nicotinamide was applied four times daily by a 21 year-old male with a mixture of comedones and papulo-pustules on his face. After ten days of treatment his face was much improved and after four weeks of such treatment, it was almost completely clear.

Example 7

A 16 year-old male with moderate severe inflammatory acne consisting of primarily papules and pustules as well as a lessor number of comedones applied a gel containing 15% carbamide peroxide and 10% nicotinamide twice daily. The patient's lesions almost completely cleared within ten (10) days but he had to discontinue the product because of excessive chapping.

Liquid dosage forms for topical administration includes acceptable emulsions, solutions and suspensions containing volatile diluents commonly used in the art, such as alcohols and glycols. Besides such diluents, topically applied compositions may also include wetting agents, emulsifying and suspending agents.

Claims

1. The use of a therapeutically effective amount of carbamide peroxide for the manufacture of a topical ointment, cream or lotion for the treatment of acne vulgaris including open and closed comedones.

2. The use of claim 1, wherein the carbamide peroxide is provided in an amount not less than 1% by volume of the inactive ingredients.

3. The use of claim 1, wherein the carbamide peroxide is provided in the range of from 1% to 15% by volume of the inactive ingredients.

4. A composition effective in decreasing the inflammatory lesions of acne vulgaris in human patients having such inflammatory lesions comprising a carrier containing a therapeutically effective amount of carbamide peroxide in combination with a therapeutically effective amount of nicotinic acid or nicotinamide.

5. The composition of claim 4, wherein the carbamide peroxide is present in an amount not less than 1% by volume of the carrier.

6. The composition of claim 4, wherein the carbamide peroxide is present in the range of from 1% to 15% by volume of the carrier.

7. The composition of claim 4, wherein nicotinamide is present in the range of from 1% to 10% by volume of the carrier.

Patentansprüche

1. Verwendung einer therapeutisch wirksamen Menge Carbamidperoxid zur Herstellung einer topischen Salbe, Creme oder Lotion für die Behandlung der Acne vulgaris mit offenen und geschlossenen Komedonen.

2. Verwendung nach Anspruch 1, dadurch gekennzeichnet, daß das Carbamidperoxid in einer Menge von mindestens 1 Vol.% der inaktiven Bestandteile verwendet wird.

3. Verwendung nach Anspruch 1, dadurch gekennzeichnet, daß das Carbamidperoxid in einer Menge von 1 bis 5 vol.% der inaktiven Bestandteile verwendet wird.

4. Zusammensetzung zum Verkleinern der entzündlichen Läsionen der Acne vulgaris bei menschlichen Patienten mit derartigen entzündlichen Läsionen, mit einem Träger, der eine therapeutisch wirksame Menge Carbamidperoxid in Kombination mit einer therapeutisch wirksamen Menge Nicotinsäure oder Nicotinamid enthält.

5. Zusammensetzung nach Anspruch 4, dadurch gekennzeichnet, daß das Carbamidperoxid in einer Menge von mindestens 1 Vol.% des Trägers vorhanden ist.

6. Zusammensetzung nach Anspruch 4, dadurch gekennzeichnet, daß das Carbamidperoxid in einer Menge von 1 bis 15 Vol.% des Trägers vorhanden ist.

7. Zusammensetzung nach Anspruch 4, dadurch gekennzeichnet, daß das Nicotinamid in einer Menge von 1 bis 10 Vol.% des Trägers vorhanden ist.

Revendications

1. Utilisation d'une quantité thérapeutiquement efficace de peroxyde de carbamide par la préparation d'une pommade, crème ou lotion topique pour le traitement de l'acné vulgaris, y compris des comédons ouverts et fermés.

2. Utilisation selon la revendication 1, dans laquelle le peroxyde de carbamide est apporté dans une proportion supérieure ou égale à 1% des ingrédients inactifs, en volume.

3. Utilisation selon la revendication 1, dans laquelle le peroxyde de carbamide est apporté dans une proportion comprise entre 1% et 15% des ingrédients inactifs, en volume.

4. Composition efficace dans la diminution des lésions inflammatoires de l'acné vulgaris chez les patients humains présentant de telles lésions inflammatoires comprenant un vecteur contenant une quantité thérapeutiquement efficace de peroxyde de carbamide en combinaison avec une quantité thérapeutiquement efficace d'acide nicotinique ou de nicotinamide.

5. Composition selon la revendication 4, dans laquelle le peroxyde de carbamide est présent dans une proportion supérieure ou égale à 1% du vecteur, en volume.

6. Composition selon la revendication 4, dans laquelle le peroxyde de carbamide est présent dans une proportion comprise entre 1% et 15% du vecteur, en volume.

7. Composition selon la revendication 4, dans laquelle le nicotinamide est présent dans une proportion comprise entre 1% et 10% du vecteur, en volume.